# EUROPEAN PATENT APPLICATION

(11) **EP 4 649 880 A1**
(43) Date of publication of application: **19.11.2025**
(21) Application number: 25174653.3
(22) Date of filing: 06.05.2025
(51) Int. Cl.: A61B 1/00, G16H 40/40, G16H 40/60

(54) **ENDOSCOPE, ENDOSCOPE MAINTENANCE SYSTEM, DISTRIBUTED ENDOSCOPE MAINTENANCE SYSTEM, AND METHODS OF COMMUNICATING DATA IN A DISTRIBUTED ENDOSCOPE MAINTENANCE SYSTEM**

(30) Priority: 14.05.2024 DE 102024113497
(71) Applicant: OLYMPUS WINTER & IBE GMBH, 22045 Hamburg (DE)
(72) Inventor: Jaskola, Sascha, 22767 Hamburg (DE); Mückner, Andreas, 21493 Schwarzenbek (DE)
(74) Representative: Noack, Andreas

(57) **Abstract**

Described herein is an endoscope with a main body and an elongate shaft,
wherein the endoscope comprises an internal memory storage element, a controller, and a communication interface, the controller being configured to receive, through the communication interface, one or more of usage-related data, reprocessing-related data, and error-related data, and to store such data on the memory storage element; and
the controller further being configured to read, from the memory storage element, such one or more of usage-related data, reprocessing-related data, and error-related data, and to output the data through the communication interface.

Also provided are endoscope maintenance systems and methods for communicating data therein.

## Description

### Field

The present disclosure relates to endoscopes and endoscope maintenance systems. More specifically, this disclosure relates to endoscopes comprising a memory storage element enabling the endoscope to act as a data transfer vehicle and the use thereof in endoscope maintenance systems.

### Background

Endoscopes are being used in the medical field since several decades. Having started as purely optical instruments, modern endoscopes often comprise integrated video cameras and associated circuitry. Such endoscopes further comprise electrical connectors for connecting the endoscope to further medical devices like camera control units (CCU).

Even endoscopes not comprising internal video cameras often carry electronic identification tags, e.g. RFID tags, for identification of the endoscope by other medical devices like endoscope reprocessing machines (ERM).

During operation and maintenance of modern endoscopes, there is often a need to communicate data.

Safe and effective reprocessing of endoscopes in ERM requires the correct setting of process definition parameters in the ERM. From time to time, updated or improved process definition parameters need to be communicated from a service computer, which may be a service computer within a facility of the producer of the ERM, to individual ERM in the field.

During reprocessing, ERM may acquire and monitor process execution parameters, to detect any deviation of process execution parameters from process definition parameters. In case of deviations, the ERM may generate an error message. Parameter deviations may be ERM related. ERM related deviations may include a reprocessing fluid not reaching a predetermined minimum temperature, a reprocessing agent not being available in a required amount, or the like. Parameter deviations may be endoscope related. Endoscope related deviations may include an endoscope failing a leakage test, an endoscope failing a channel patency test, or the like. In case of ERM related deviations, it may be necessary to communicate data related to the deviation with the producer of the ERM for assisting correction of the fault. In case of endoscope related deviations, it may be necessary to communicate data related to the deviation with a producer of the endoscope for assisting repair of the endoscope.

Operation of an endoscope together with a CCU may require correct setting of several operation parameters of the endoscope within the CCU. This may include white balance parameters, brightness sensitivity parameters, or the like. From time to time, it may be necessary to communicate updated parameters to the CCU.

During operation of an endoscope together with a CCU, the CCU may monitor correct operation of the endoscope. For example, the CCU may detect image-related errors like defunct pixels in a video image acquired by the endoscope, blurriness of a video image acquired by the endoscope, insufficient brightness of a video image acquired by the endoscope, or the like. In case of errors detected by the CCU, it may be necessary to communicate data related to the error to a manufacturer of the endoscope to assist repair of the endoscope.

Some modern medical devices like ERM or CCU comprise cloud connectivity to enable data communication of the respective medical devices with service computers of their respective manufacturers. However, far from all medical devices comprise cloud connectivity. Further, such cloud connectivity is of limited use in situations where, for example, data related to an endoscope related parameter deviation needs to be communicated from an ERM to the manufacturer of the endoscope, or data related to an endoscope error needs to be communicated from a CCU to the manufacturer of the endoscope, or the like.

The DE 20 2006 008 027 U1 suggests an endoscope with an integrated measuring device for storing and transmitting temperature or acceleration events outward.

It is an object of the disclosure to provide for improved ways of communicating data between endoscopes, service computers, and/or medical devices.

### Summary of the disclosure

The present disclosure provides an endoscope with a main body and an elongate shaft,
wherein the endoscope comprises an internal memory storage element, a controller, and a communication interface, the controller being configured to receive, through the communication interface, one or more of usage-related data, reprocessing-related data, and error-related data, and to store such data on the memory storage element; and
the controller further being configured to read, from the memory storage element, such one or more of usage-related data, reprocessing-related data, and error-related data, and to output the data through the communication interface. An endoscope according to this disclosure is capable of serving as a vehicle for communicating data within a local or distributed endoscope maintenance system.

The communication interface may comprise a wired communication interface, a wireless communication interface, or both. Wired communication interfaces may include, but are not limited to, RS232 interfaces, USB interfaces, ethernet interfaces, customized interfaces presently used for communication between endoscopes and CCUs, or the like. Wireless interfaces may include, but are not limited to, WiFi interfaces, Bluetooth interfaces, IR interfaces, RFID interfaces, NFC interfaces, or the like.

Usage-related data may comprise one or more of a total use time of the endoscope, one or more operational parameters selected for use with the endoscope, one or more procedures performed with the endoscope, an instrument ID of a medical device used together with the endoscope, or the like.

Reprocessing-related data may comprise one or more of an instrument ID of a reprocessing device in which the endoscope has been processed, process parameters of a reprocessing process applied to the endoscope, or the like.

Error-related data may comprise one or more of data related to image-related errors, reprocessing process errors, patency check errors, leakage test errors, or the like.

The disclosure further provides an endoscope maintenance system, comprising at least one endoscope according to the above description; at least one medical device configured to communicate with the controller of the endoscope through the communication interface; and a service computer, the service computer comprising a service interface for communication with the controller of the endoscope through the communication interface. The at least one medical device may include, but is not limited to, an ERM, a CCU, or both. The service computer may be any standard or medical grade computer comprising a processor, memory, and input/output devices. Software may be stored on the memory for execution by the processor. The software may comprise an operating system like Microsoft Windows, Mac OS, Linux, or the like. The software may further comprise software for management of data associated with one or more endoscopes, one or more ERM, one or more CCUs, or other medical devices. The service computer may be configured to read, through the service interface, data stored on the memory storage element of the endoscope. The service computer may be configured to write, through the service interface, data to the memory storage element of the endoscope.

Throughout this disclosure, the term "maintenance system" is used to describe systems which enable predictive and reactive maintenance of endoscopes and/or repair of endoscopes. Such systems may include elements which are not exclusively used for the purpose of maintenance, but also for the purpose of general operation of endoscopes. For example, CCUs and ERMs may both be used in operation and in maintenance of endoscopes.

In certain embodiments, the disclosure provides a method of distributing data in the endoscope maintenance system described above, comprising preparing data to be distributed to the at least one medical device using the service computer; connecting the endoscope to the service computer using the communication interface and the service interface; transferring the data from the service computer to the controller of the endoscope through the communication interface and the service interface; storing the data on the memory storage element of the endoscope; disconnecting the endoscope from the service computer; connecting the endoscope to at least one medical device using the communication interface; reading the data from the memory storage element using the controller; and transferring the data from the controller to the medical device.

Data to be distributed to one or more medical devices may include updated reprocessing parameters to be distributed to one or more ERMs, updated image processing software to be distributed to one or more CCUs, or the like.

In other embodiments, the disclosure provides a method of communicating data in the endoscope maintenance system described herein, comprising preparing data to be communicated to a service computer using at least one medical device; connecting the endoscope to at least one medical device using the communication interface; transferring the data from the medical device to the controller of the endoscope through the communication interface; storing the data on the memory storage element of the endoscope; disconnecting the endoscope from the medical device; connecting the endoscope to the service computer using the communication interface and the service interface; reading the data from the memory storage element using the controller; and transferring the data from the controller to the service computer.

Data to be communicated from one or more medical devices to a service computer may include usage-related data prepared by a CCU, reprocessing log data prepared by an ERM, or error-related data prepared by either a CCU or an ERM.

In some embodiments, the disclosure provides a distributed endoscope maintenance system, comprising at least one endoscope according to the above description; a service computer, the service computer having cloud connectivity; at least one first medical device configured to connect with the at least one endoscope through the communication interface, the first medical device not having cloud connectivity; and at least one second medical device configured to connect with the at least one endoscope through the communication interface, the second medical device having cloud connectivity.

In further embodiments, the present disclosure provides a method of communicating data in a distributed endoscope maintenance system as described above, comprising preparing data to be distributed to a medical device using the service computer; communicating the data from the service computer to at least one second medical device using cloud connectivity; connecting the endoscope to the at least one second medical device using the communication interface; transferring the data from the second medical device to the controller of the endoscope through the communication interface; storing the data on the memory storage element of the endoscope; disconnecting the endoscope from the second medical device; connecting the endoscope to at least one first medical device using the communication interface; reading the data from the memory storage element using the controller; and transferring the data from the controller to the first medical device.

In even further embodiments, the present disclosure provides a method of communicating data in a distributed endoscope maintenance system as described herein, comprising: preparing data to be communicated to a service computer using at least one first medical device; connecting the endoscope to at least one first medical device using the communication interface; transferring the data from the first medical device to the controller of the endoscope through the communication interface; storing the data on the memory storage element of the endoscope; disconnecting the endoscope from the first medical device; connecting the endoscope to at least one second medical device using the communication interface; reading the data from the memory storage element using the controller; transferring the data from the controller to the second medical device; and communicating the data from the at least one second medical device to the service computer using cloud connectivity.

### Brief description of the drawings

Exemplary embodiments of the present disclosure are hereafter further described at the hand of drawings. The embodiments and drawings herein are only provided for easier understanding of the disclosure, and are not meant to be limiting the scope of the appending claims. The drawings may be simplified, and are not necessarily drawn to scale.
Fig. 1 shows a schematic view of an endoscope;
Fig. 2 shows a schematic view of a further endoscope;
Fig. 3 shows an endoscope maintenance system;
Figs. 4a,b show flowcharts of methods for distributing or communicating data;
Fig. 5 shows a distributed endoscope maintenance system;
Figs. 6a,b show flowcharts of further methods for distributing or communicating data.

### Detailed description

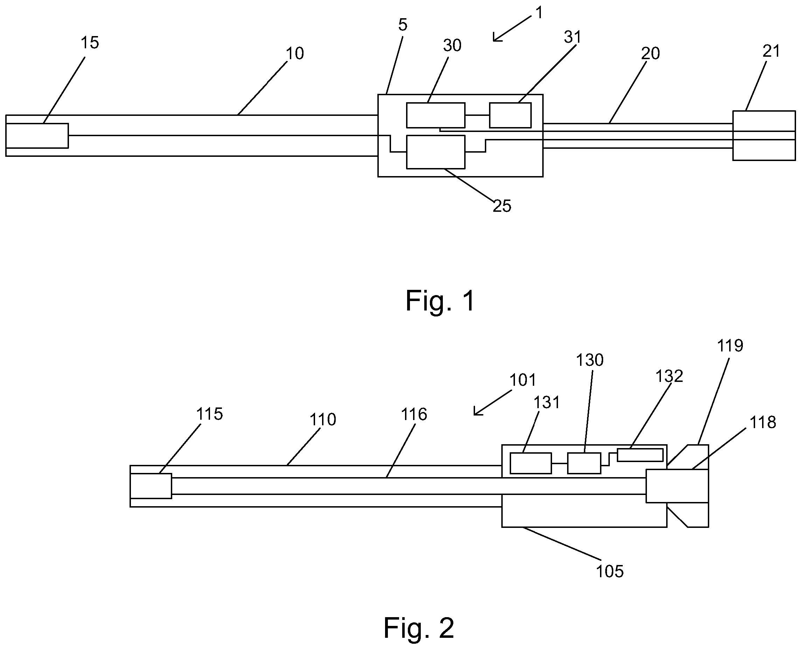

Fig. 1 shows a schematic view of an endoscope 1, which is a video endoscope. The endoscope 1 comprises a main body 5 and an elongated shaft 10 extending distally therefrom. At the distalmost end of the shaft, a video camera 15 is disposed, through which images of an anatomical structure of interest may be acquired. A connection cable 20 is attached to a proximal end of the main body 5 and terminates in a connector plug 21. The connector plug 21 serves to connect the endoscope 1 to an external medical device like a CCU (not shown).

The video camera 15 is connected to video preprocessing circuitry 25 which is provided in the endoscope 1. The video preprocessing circuitry is connected to the connector plug 21. While Fig. 1 shows connections between the video camera 15, the video preprocessing circuitry 25, and the connector plug 21 as single lines, it is appreciated that such connections will normally employ a plurality of physical connection lines carrying electric supply voltage, synchronization signals, video signals, and the like.

Inside the endoscope 1 there is further provided a controller 30, which may be a microcontroller, and a memory storage element 31, which are connected to each other. The memory storage element 31 may be a static memory element capable of maintaining the data without need for permanent power supply. The memory storage may be a flash memory. The microcontroller 30 is further connected to the plug 21, forming a wired communication interface. Again, connections between the controller 30, the memory storage element 31, and the plug are shown as single lines, but may typically employ several physical lines.

Through the wired communication interface, the controller 30 may communicate with external medical devices like a CCU, to either receive data from the external medical device and write such data to the memory storage element 31, or to read data from the memory storage element 31 and send such data to the external medical device. The data may include, but is not limited to, use-related data, reprocessing-related data, or error-related data. As explained further below, data exchanged through the communication interface between the controller 30 of the endoscope 1 and the external medical device need not directly be related to the endoscope 1 or the external medical device, but may data related to a further device where the endoscope 1 acts as a data transfer vehicle.

Fig. 2 shows a further endoscope 101. Other than the endoscope 1, the endoscope 101 is an optical endoscope. The endoscope 101 comprises a main body 105 and an elongate shaft 110. At the distal end of the shaft 110, an objective lens system 115 is disposed, through which an image of an anatomical structure of interest may be formed. An optical image guide 116 transfers the image formed by the objective lens system 110 to an eyepiece lens system 118 at the proximal end of the main body 110. An eyepiece cone 119 enables direct viewing of the transferred image with the naked eye, or connection of an external video camera head for converting the image into video image signals. The optical image guide 116 may be an optical fiber bundle. Optical fiber bundle image guides are often used in endoscopes having a flexible or semi-rigid shaft. In other embodiments, the optical image guide may be a relay lens system. Relay lens image guides are often employed in endoscopes having a rigid shaft.

Similar to endoscope 1, the endoscope 101 comprises a controller 130 and a memory storage element 131. Other than in the endoscope 1, the controller 130 of endoscope 101 is further connected to a wireless communication interface 132, which may by any of a WiFi interface, a Bluetooth interface, am RFID interface, or any other suitable wireless interface. The endoscope 101 may further comprise an internal energy source like a primary or secondary battery. A primary battery may be dimensioned to provide sufficient supply current for an expected lifetime of the endoscope 101. A secondary battery may be configured to be recharged when depleted. Recharging may be effected using suitable contact-free inductive, capacitive, or resonant charging circuitry (not shown). In some embodiments, recharging of a secondary battery may be effected using photovoltaic cells irradiated by a fraction of light provided to the endoscope 101 for illumination of the imaging target. In other embodiments, the energy source may be a radionuclide battery like recently introduced by Betavolt Technology.

Through the wireless communication interface 132, the controller 130 may communicate with external medical devices like an ERM (not shown), to either receive data from the external medical device and write such data to the memory storage element 131, or to read data from the memory storage element 131 and send such data to the external medical device. Again, the data may include, but is not limited to, use-related data, reprocessing-related data, or error-related data.

While Figs. 1 and 2 show a wired communication interface in connection with a video endoscope, and a wireless communication interface in connection with an optical endoscope, the disclosure is not limited thereto. In other embodiments not shown, a video endoscope may be equipped with a wireless communication interface, an optical endoscope may be equipped with a wired communication interface, or any kind of endoscope may be equipped with both a wired and a wireless communication interface.

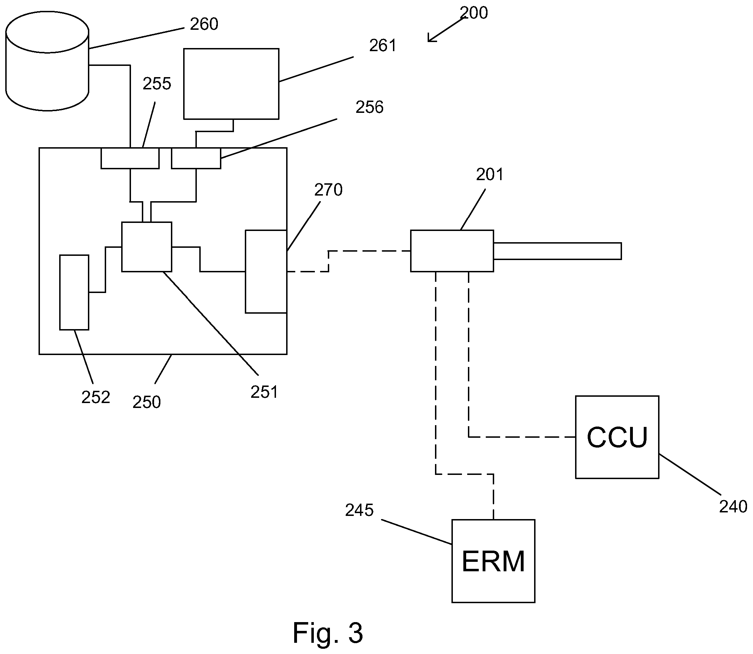

Fig. 3 shows an endoscope maintenance system 200. The endoscope maintenance system 200 comprises at least one endoscope 201, which may be the endoscope 1 of Fig. 1 or the endoscope 101 of Fig. 2. The endoscope maintenance system 200 further comprises medical devices 240, which is a CCU, and a medical device 245, which is an ERM. CCU, also known as camera controllers or endoscope controllers, are medical devices specifically designed for controlling endoscopes, and may be obtained under the device name OTV-S400 from Olympus Europa SE & Co. KG, Wendenstraße 20, 20097 Hamburg, Germany. ERM are medical devices specifically designed for reprocessing of endoscopes and other surgical instruments. Reprocessing may include cleaning, disinfection, and sterilization. ERM may be obtained under the product name ETD from Olympus Europa SE & Co. KG, Wendenstraße 20, 20097 Hamburg, Germany. Each of the CCU 240 and the ERM 245 are configured to connect with the controller of the endoscope 201 through the communication interface of the endoscope 201.

The endoscope maintenance system 200 further comprises a service computer 250, which may be a standard or medical grade general purpose computer. The service computer comprises a processor 251, which may be a single- or multi-core processor, a memory element 252, which may include a random-access memory (RAM), a read only memory (ROM), a Flash memory, or a combination thereof. The service computer 250 may comprise a network interface 255 and USB interface 256. The network interface 255, which may include an ethernet interface, may connect the service computer to a database 260. The USB interface 256 may connect the service computer 250 to input/output devices 261, which may include one or more of a keyboard, a computer mouse, a trackpad, a monitor, and a touch screen device.

The service computer 250 further comprises a service interface 270 configured to connect with the communication interface (not shown in Fig. 3) of the endoscope 201. Depending on the type of the communication interface, a connection between the service interface 270 and the communication interface may be a wired connection or a wireless connection.

Through the connection between the service interface 370 and the communication interface, the service computer 250 can communicate with the controller of the endoscope 201 to read data from the memory storage element of the endoscope 201, write data to the memory storage element of the endoscope 201, or both.

The broken lines leading from the endoscope 201 to the CCU 240, the ERM 245, and the service interface 270, indicate that the respective devices may be variably connected to and disconnected from each other.

For example, the endoscope 201 may be a new endoscope type introduced to the market, and ERMs in the field may not have appropriate reprocessing parameters stored in their local memories. The new reprocessing parameters may be provided by the manufacturer of the endoscope, and be prepared on the service computer 250 for distribution. Such preparation may involve bringing the data into a specific data format, compressing the data, encrypting the data, electronically signing the data, or the like.

The endoscope 201 is then connected to the service interface 270 of the service computer 250, and the processor 251 may transfer the prepared data to the controller of the endoscope 201, which then writes the data to the memory storage element of the endoscope 201. After that, the endoscope 201 is disconnected from the service interface 270.

In the field, the endoscope 201 will then have to be reprocessed in the ERM 245. Therefore, the communication interface of the endoscope 201 is connected to the ERM. The controller of the endoscope 201 then reads the data from the memory storage element and transfers the data to the ERM. The ERM, using suitable processing hardware provided in the ERM, uses the data to set appropriate reprocessing parameters for secure and effective reprocessing of the endoscope 201.

In another example, when an improved imaging method is developed by the producer of the CCU 240, corresponding software needs to be distributed to CCUs in the field. Distribution of the software can be effected by using the same method as described above for distribution of new reprocessing parameters.

In other examples, endoscope 201 may be sent to the manufacturer thereof for servicing or repair.

Servicing may include thoroughly checking the endoscope 201 for any damages after a predefined number of uses or at a predetermined age. The manufacturer may want to know how many times the endoscope 201 has been used since production or since the most recent servicing. To easily and reliably providing that information, the CCU 240 (or other CCUs with which the endoscope 201 is used) are connected to the communication interface of the endoscope, and send use-related data to the controller of the endoscope 201. The controller then writes the data to the memory storage element of the endoscope 201. When received by the manufacturer, the endoscope 201 is connected to the service computer 250 through the service interface 270, and the respective use-related data is read out and transferred to the service computer. The service computer 250 may use the data to update database 260, in which use-related data of a plurality of endoscopes may be stored.

Repair may include replacing damaged components of endoscope 201 after an error has occurred in the field. Errors may be reprocessing-related errors detected by an ERM, like leakage or blocked channels. Errors may be imaging -related errors detected by a CCU, like blurry image, defunct pixels, or the like. The medical device detecting the error can use the communication interface of the endoscope 201 to transmit the error-related data to the controller of the endoscope 201, which then writes such information to the memory storage element of the endoscope 201. When received by the manufacturer, the endoscope 201 is connected to the service computer 250 through the service interface 270, and the respective error-related data is read out and transferred to the service computer. The service computer 250 may use the error-related data to identify and retrieve information in the database 260, which may be used to assist in repairing the endoscope 201.

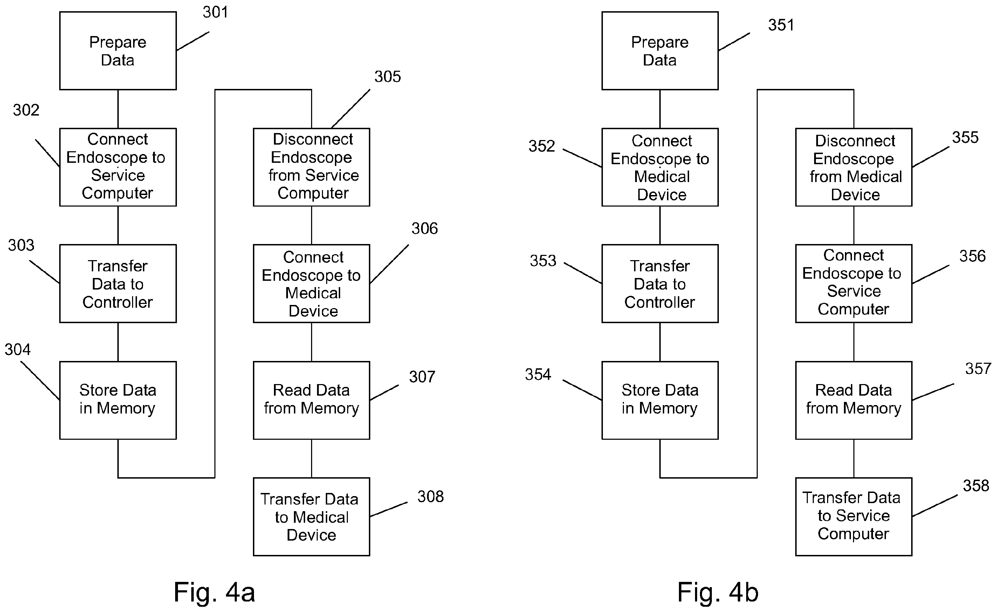

Figs. 4a and 4b show schematic flow charts of methods for distributing or communicating data in an endoscope maintenance system like the system 200.

Fig 4a shows a method for distributing data from a service computer to one or more medical devices. In a first step 301, data for distribution is prepared on the service computer. In step 302, the endoscope is connected to the service computer using the communication interface and the service interface. In step 303, the data is transferred to the controller of the endoscope. In step 304, the controller writes the data to the memory storage element. In step 305 the endoscope is disconnected from the service computer. In step 306, the endoscope is connected to a medical device. In step 307, the controller reads the data from the memory storage element, and in step 308 the data is transferred to the medical device.

Fig 4b shows a method for communicating data from a medical device to one a service computer. In a first step 351, data is prepared on the medical device. In step 352, the endoscope is connected to the medical device using the communication interface. In step 353, the data is transferred to the controller of the endoscope. In step 354, the controller writes the data to the memory storage element. In step 355 the endoscope is disconnected from the medical device. In step 356, the endoscope is connected to a service computer using the communication interface and the service interface. In step 357, the controller reads the data from the memory storage element, and in step 358 the data is transferred to the service computer.

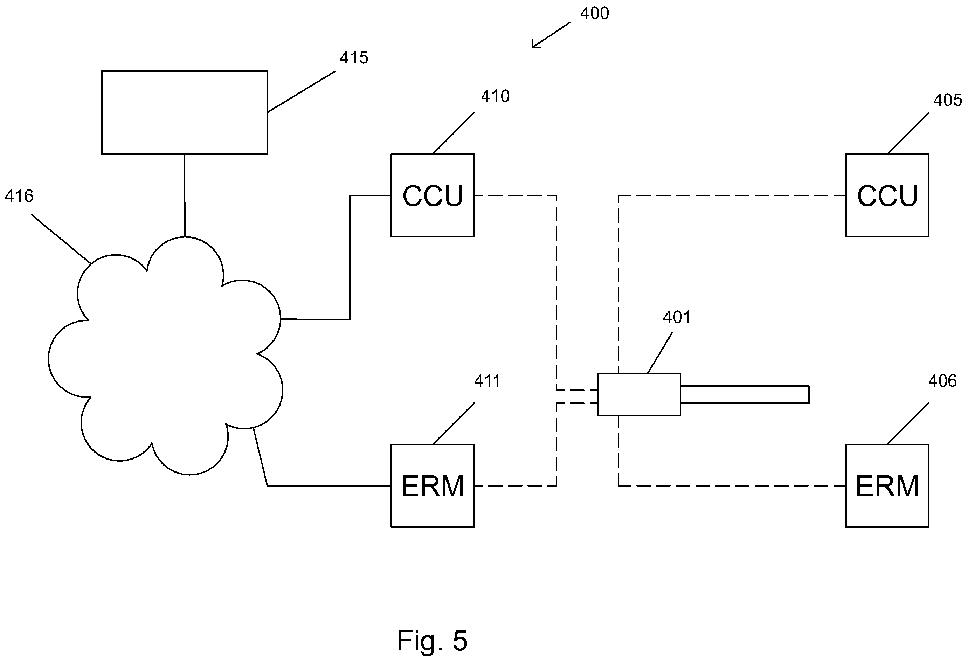

Fig. 5 shows a schematic view of a distributed endoscope maintenance system 400. The system 400 comprises an endoscope 401, which may be similar to the endoscope 1 or 101 and is not further described here for brevity.

The system 400 further comprises first medical devices 405, 406, which may be a CCU and an ERM. The medical devices 405, 406 are stand-alone devices not having any cloud connectivity.

The system 400 further comprises second medical devices 410, 411, which again may be a CCU and an ERM. Other than the first medical devices 405, 406, the second medical devices 410, 411 are equipped with cloud connectivity to communicate with a remote service computer 415 through a network 416. The service computer 415 may be similar to service computer 250, but may not necessarily have a service interface.

The term "cloud connectivity" is herein used to describe the capability of respective devices to communicate with other devices though a network like network 416. While the term "cloud" is generally used to describe decentralized services in which a service receiver doe not necessarily know or need to know where a service provider is located, the term "cloud connectivity" as used herein is also meant to cover setups where a network connection is made between distinct known locations, like between a medical facility using one or more of the medical devices 405, 406, 410, 411 and a manufacturer of these medical devices providing a dedicated service computer 415.

Medical devices 410, 411 can always use the cloud connectivity to exchange use-related data, error-related data, or other data with the service computer 415, while medical devices 405, 406 cannot. In the distributed endoscope maintenance system 400, the endoscope 401 is used as a data transfer vehicle, as the endoscope 401 can exchange data with all medical devices 405, 406, 410, 411 through the communication interface.

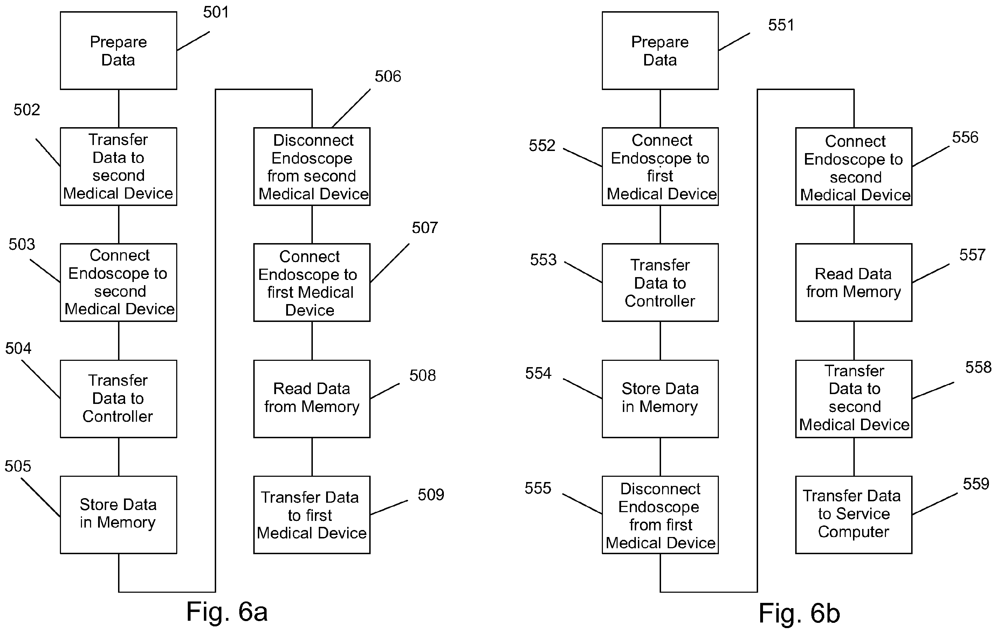

Methods for distributing or communicating data in the distributed endoscope maintenance system 401 are described in the following at hand of schematic flowcharts in Figs. 6a, 6b.

Fig. 6a shows a method to distribute data from service computer 415 to one or more first medical devices 405, 406, which do not comprise cloud connectivity. In step 501, the data is prepared on the service computer 415. In step 502, the data is transferred to at least one second medical device 410, 411, using cloud connectivity. In step 503, the endoscope 401 is connected to the second medical device 410, 411 using the communication interface. In step 504, the data is transferred to the controller of the endoscope 401. In step 505, the data is written to the memory storage element of the endoscope 401. In step 506, the endoscope 401 is disconnected from the second medical device 410, 411. In step 507, the endoscope 401 is connected to the first medical device 405, 406 using the communication interface. In step 508, the data is read from the memory storage element by the controller. In step 509, the data is transferred to the first medical device 405, 406.

Fig. 6b shows a method to communicate data from one or more first medical devices 405, 406, which do not comprise cloud connectivity, to a service computer 415. In step 551, the data is prepared on the first medical device 405, 406. In step 552, the endoscope 401 is connected to the first medical device 405, 406 using the communication interface. In step 553, the data is transferred to the controller of the endoscope 401. In step 554, the data is written to the memory storage element of the endoscope 401. In step 555, the endoscope 401 is disconnected from the first medical device 405, 406. In step 556, the endoscope 401 is connected to the second medical device 410, 411 using the communication interface. In step 557, the data is read from the memory storage element by the controller. In step 558, the data is transferred to at least one second medical device 410, 411. In step 559, the data is transferred to the service computer 415, using cloud connectivity.

As can be seen from the above description, an endoscope as described herein can serve as a data transfer vehicle to enable data exchange between a service computer and medical devices. For example, larger medical facilities use a large number of endoscopes, which are repeatedly connected to different medical devices like CCUs and ERMs. Using the systems and methods described herein, in may be sufficient to provide a limited number of medical devices with cloud connectivity, and to use the endoscopes as data transfer vehicles, to efficiently distribute and/or communicate up-to-date data between all medical devices and a service computer having cloud connectivity.

## Claims

1. An endoscope with a main body (5, 105) and an elongate shaft (10, 110),
wherein the endoscope comprises an internal memory storage element (31, 131), a controller (30, 130), and a communication interface (132),
the controller (30, 130) being configured to receive, through the communication interface (132), one or more of usage-related data, reprocessing-related data, and error-related data, and to store such data on the memory storage element (31, 131); and
the controller (30, 130) further being configured to read, from the memory storage element (31, 131), such one or more of usage-related data, reprocessing-related data, and error-related data, and to output the data through the communication interface (132).

2. The endoscope of claim 1, wherein the communication interface (132) comprises a wired communication interface, a wireless communication interface (132), or both.

3. The endoscope of claim 1 or 2, wherein the usage-related data comprises one or more of:
a total use time of the endoscope, one or more operational parameters selected for use with the endoscope, one or more procedures performed with the endoscope, an instrument ID of a medical device used together with the endoscope, or the like.

4. The endoscope of any of claims 1 to 3, wherein the reprocessing-related data comprises one or more of:
an instrument ID of a reprocessing device, in which the endoscope has been processed, process parameters of a reprocessing process applied to the endoscope, or the like.

5. The endoscope of any of claims 1 to 4, wherein the error-related data comprises one or more of:
data related to video communication errors, reprocessing process errors, patency check errors, leakage test errors, or the like.

6. An endoscope maintenance system, comprising:
- at least one endoscope (201) according to any one of claims 1 to 5,
- at least one medical device (240, 245) configured to communicate with the controller of the endoscope (201) through the communication interface; and
- a service computer (250), the service computer comprising a service interface (270) for communication with the controller of the endoscope (201) through the communication interface.

7. A method of communicating data in the endoscope maintenance system of claim 6, comprising:
- preparing data to be distributed to the at least one medical device using the service computer;
- connecting the endoscope to the service computer using the communication interface and the service interface;
- transferring the data from the service computer to the controller of the endoscope through the communication interface and the service interface;
- storing the data on the memory storage element of the endoscope;
- disconnecting the endoscope from the service computer;
connecting the endoscope to at least one medical device using the communication interface;
- reading the data from the memory storage element using the controller; and
- transferring the data from the controller to the medical device.

8. A method of communicating data in the endoscope maintenance system of claim 6, comprising:
- preparing data to be communicated to a service computer using at least one medical device;
- connecting the endoscope to at least one medical device using the communication interface;
- transferring the data from the medical device to the controller of the endoscope through the communication interface;
- storing the data on the memory storage element of the endoscope;
- disconnecting the endoscope from the medical device;
- connecting the endoscope to the service computer using the communication interface and the service interface;
- reading the data from the memory storage element using the controller; and
- transferring the data from the controller to the service computer.

9. A distributed endoscope maintenance system, comprising:
- at least one endoscope (401) according to one of claims 1 to 5;
- a service computer (415), the service computer having cloud connectivity;
- at least one first medical device (405, 406) configured to connect with the at least one endoscope (401) through the communication interface, the first medical device (405, 506) not having cloud connectivity; and
- at least one second medical device (410, 411) configured to connect with the at least one endoscope (401) through the communication interface, the second medical device (410, 411) having cloud connectivity.

10. A method of communicating data in the distributed endoscope maintenance system of claim 9, comprising:
- preparing data to be distributed to a medical device using the service computer;
- communicating the data from the service computer to at least one second medical device using cloud connectivity;
- connecting the endoscope to the at least one second medical device using the communication interface;
- transferring the data from the second medical device to the controller of the endoscope through the communication interface;
- storing the data on the memory storage element of the endoscope;
- disconnecting the endoscope from the second medical device; connecting the endoscope to at least one first medical device using the communication interface;
- reading the data from the memory storage element using the controller; and
- transferring the data from the controller to the first medical device.

11. A method of communicating data in the distributed endoscope maintenance system of claim 9, comprising:
- preparing data to be communicated to a service computer using at least one first medical device;
- connecting the endoscope to at least one first medical device using the communication interface;
- transferring the data from the first medical device to the controller of the endoscope through the communication interface;
- storing the data on the memory storage element of the endoscope;
- disconnecting the endoscope from the first medical device;
- connecting the endoscope to at least one second medical device using the communication interface;
- reading the data from the memory storage element using the controller;
- transferring the data from the controller to the second medical device; and
- communicating the data from the at least one second medical device to the service computer using cloud connectivity.
